# EUROPEAN PATENT SPECIFICATION

(11) **EP 4 370 494 B1**
(45) Date of publication and mention of the grant of the patent: **09.07.2025**
(21) Application number: 22760778.5
(22) Date of filing: 12.07.2022
(51) Int. Cl.: C07C 37/82, C07C 39/11, B01D 15/08

(54) **PROCESS FOR OBTAINING CONCENTRATED HYDROXYTYROSOL (HT) EXTRACTS**
VERFAHREN ZUR GEWINNUNG VON KONZENTRIERTEN HYDROXYTYROSOLEXTRAKTEN
PROCÉDÉ POUR OBTENIR DES EXTRAITS CONCENTRÉS D'HYDROXYTYROSOL (HT)

(30) Priority: 15.07.2021 CL 20211885
(43) Date of publication of application: 22.05.2024
(73) Proprietor: American Bioprocess Limitada, Valparaíso (CL)
(72) Inventor: MANCILLA VILLALOBOS, Rodrigo Alejandro, Valparaíso (CL); ARAVENA CONTRERAS, Raúl Ignacio, Providencia Santiago (CL)
(74) Representative: Clarke, Modet y Cía., S.L.
(86) International application number: PCT/IB2022/056431
(87) International publication number: WO 2023/285969

(56) References cited:
- WO-A1-2008/090460

## Description

### FIELD OF THE INVENTION

A process for the purification of extracts with low hydroxytyrosol (HT) concentration without the use of organic solvents is disclosed in order to obtain concentrated HT products.

Hydroxytyrosol (HT) is a molecule which has a series of health benefits such as hypocholesterolemic, antioxidant, depigmenting, antiinflammatory, anticancer, neuroprotective, among others. It has also been proposed that concentrated hydroxytyrosol extracts could have benefits in animal feed (for example, in salmon), and works as an antioxidant in food products (for example, meat products and oils) and as an antimicrobial.

The molecular structure of hydroxytyrosol (HT) is:

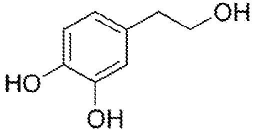

Hydroxytyrosol can be chemically synthesized or recovered from natural sources. The increasing consumer demand for natural products instead of chemical derivatives makes interesting to obtain extracts concentrated in HT from natural sources. Currently, the main sources of natural HT are olives and by-products derived thereof. Particularly, HT extracts are currently manufactured mainly from by-products of the olive oil industry (olive oil waste, olive oil lees) and olive leaves. Olive oil waste is the most abundant by-product in the olive oil industry, which is obtained after removing the oil from the fruit (2-phase process), and is mainly constituted of vegetation water, process water, and solid derived from the fruit as the pit and the pulp.

Olive oil waste is rich in HT presenting concentrations as high as 2% dry base (d.b). Olive leaves do not have a high HT concetration, but they do have a high oleuropein concentration. In this sense, it is worth mentioning that oleuropein can be hydrolyzed to produce HT by using acids or enzymes, which has raised interest in olive leaves as an HT source even when its HT concentration is low. However, concentration of hydroxytyrosol in olive oil waste and concentration of oleuropein in leaves are quite variable depending on a series of factors such as species, culture conditions, storage time, harvest time, among others. Additionally, several compounds harmful for human consumption are found in olive oil waste and leaves. Therefore, developing methods for extracting and purifying HT for the production of concentrated and standardized extracts is needed. Currently, companies in the field commercialize products concentrated in hydroxytyrosol with concentrations that range from 3 to 40% of HT on a dry basis (d.b), which are mainly used in food supplements, functional foods and cosmetics.

The present patent application proposes a process for obtaining concentrated HT extracts using extracts with low hydroxytrosol concentration (less than 5% HT d.b) derived from the olive industry as raw material, without the use of organic solvents and reaching HT recovery greater than 50%. The concentrated extracts present final purities between 10-40% (d.b) of HT.

As previously mentioned, it is commercially interesting to produce HT extracts with purities between 5-35% (d.b). Thus, processes which leads to HT extracts with purities in this range from extracts with low HT concentration (less than 5% HT d.b) are required. These processes must also exhibit high recovery yields, since increasing purity at the expense of low recovery yields negatively impacts the investment and the production costs associated with the process. The process disclosed in the present invention is performed using only one purification step. One purification step reduces the complexity of the process and decreases costs associated with investment and operation, while at the same time products with HT purities between 10-40% (d.b) and HT recovery yields higher than 50% are reached. Additionally, the process does not require the use of organic solvents, which presents a benefit compared to other processes, considering the added value of natural products obtained without the use of organic solvents.

### STATE OF THE ART

In particular, the process of the present invention leads to extracts with at least 10% HT from extracts containing less than 5% HT derived from the olive industry reaching HT recovery yields higher than 50%. Organic solvents are not used in the process and only one purification step with resins is performed.

Several solutions have been found in the state of the art that partially solve the technical problem proposed. PCT application WO08142178 discloses a method for obtaining extracts with a high content of hydroxytyrosol from olive by-products. In brief, this document discloses a method that includes the steps of: extraction of phenolic compounds from the olive vegetation water or from a specific concentrated extract using a mixture of appropriate solvents; separation of the organic phase containing the soluble antioxidant polyphenolic compounds; treating the organic phase with a XAD resin. Finally, the polyphenolic compounds retained in the resin are recovered with water, giving an extract containing at least 40% of hydroxytyrosol.

Patent EP1582512 discloses a process for obtaining hydroxytyrosol from olive leaf extracts following steps of (a) subjecting olive leaf extracts to hydrolysis to obtain an aqueous mixture rich in hydroxytyrosol, (b) mixing the intermediate obtained with a macroporous resin to adsorb hydroxytyrosol in said matrix and maintain the undesirable by-products within the aqueous phase, and finally (c) separating the matrix from the aqueous phase and desorbing the hydroxytyrosol using a suitable solvent.

In addition, patent application WO13007850 A1 discloses a method for the sequential production of extracts with a high concentration of hydroxytyrosol in order to obtain a mixture of hydroxytyrosol and 3,4-dihydroxyphenylglycol (DHFG), and hydroxytyrosyl acetate. The method comprises treating a source of hydroxytyrosol such as olive and olive by-products in at least one chromatography column, using a mixture of at least two ionic resins, preferably an anionic resin with a cationic resin. In a first phase, a liquid rich in HT and/or DHFG is obtained, which is used as raw material for extraction and purification of phenolic compounds. In a second phase, an extract enriched in hydroxytyrosol and other simple phenolic compounds is obtained, a bioactive mixture of HT and DHFG, and hydroxytyrosyl acetate is formed at the same time. In this phase, the raw material is subjected to a chromatographic column containing a mixture of anionic exchange resins with at least 2% of a cationic exchange resin, and elution is carried out using water and ethyl acetate to obtain 3 fractions. Finally, in the third phase, the fractions obtained in phase 2 can be subjected to additional purification steps to obtain high purity hydroxytyrosol, DHFG, thereof mixtures, and hydroxytyrosyl acetate.

WO 2008/090460 A1 discloses the preparation of an aqueous extract from dry olive waste using demineralized water and sulphuric acid. Said crude aqueous extract is contacted with an ion exchange resin of the anionic type, previously activated by means of acetate cycle. The resin is eluted with demineralized water in order to obtain concentrated hydroxytyrosol.

None of the prior art documents solves the problem of the technique as cited in the present patent application, where the process of the present invention lead to obtain extracts with at least 10% hydroxytyrosol from low HT concentration extracts, meaning containing less than 5% HT derived from the olive industry, without using organic solvents, reaching high yields (higher than 50%), and using only one purification step with only one type of resin.

### DESCRIPTION OF THE INVENTION

The present invention discloses a purification process of hydroxytyrosol (HT) from extracts derived from the olive industry that contain less than 5% HT. The process lead to HT extracts with purities from 10 to 40% in one-single step by using a weak base anion resins and without using organic solvents.

The process of the present invention comprises the following steps:
a) adjusting the pH of an extract with low HT concentration to a pH value less than 7 using a pH regulator;
b) contacting the extract with low HT concentration with a weak base anion exchange resin of a polystyrene, styrene, divinylbenzene, polydivinylbenzene, polystyrene-DVB, or styrene-DVB matrix;
c) eluting the resin using hot water at a temperature between 50-100°C to obtain a purified HT extract.

The pretreated extract can be any extract containing less than 5% HT obtained from residues or by-products of the olive industry, including, but not limited to, olive oil waste, leaves, pits and brine.

To adjust the pH of the extract in step (a), any known pH regulator can be used, such as, but not limited to, NaOH, KOH, Ca(OH)₂, Mg(OH)₂, Na₂CO₃, K₂CO₃, NaHCO₃, KHCO₃, NH₄OH, CaOH, MgO, HCl, acetic acid, citric acid, ascorbic acid, butyric acid, lactic acid, propionic acid, tartaric acid, oxalic acid, phosphoric acid, sulfuric acid, malic acid, succinic acid, sodium lactate, potassium lactate, sodium acetate, potassium acetate, sodium citrate, potassium citrate, sodium tartrate, potassium tartrate, sodium phosphate, potassium phosphate, sodium sulfate, potassium sulfate, sodium malate, potassium malate or mixtures thereof.

The resin used in step (b) has its functional groups either neutral (uncharged) or protonated (charged). To obtain neutral functional groups any base can be used. Fur example, but limited to them, NaOH, KOH, Ca(OH)₂, Na₂CO₃, NH₄OH or mixtures thereof can be used. Any acid can be used to protonate the functional groups. For example, but not limited to them, HCl, acetic acid, citric acid, ascorbic acid, butyric acid, actic acid, propionic acid, tartaric acid, oxalic acid, phosphoric acid, sulfuric acid, malic acid, acid succinic or mixtures thereof can be used.

For the execution of step (b), HT loads in the resin between 1 and 15 mg HT/g of the resin, and more preferably between 2 and 10 mg of HT/g of the resin, with HT recovery higher than 50%, and more preferably between 55 and 90%, are achieved during the adsorption process.

The elution of the resin in step (c) must be carried out with hot water at a temperature between 50-100°C, optionally an acid can be added to work at a pH lower than 7 to avoid the degradation of HT. The acid used can be selected from hydrochloric acid, acetic acid, citric acid, ascorbic acid, butyric acid, lactic acid, propionic acid, tartaric acid, oxalic acid, phosphoric acid, sulfuric acid, malic acid, succinic acid or mixtures thereof.

For the execution of step (c), resin to water (R/W) ratios must be used that lead, once the elution is completed, to recover at least 50% of the HT originally present in the extract while a final HT purity of at least 10% in the eluted liquid is reached. More preferably, HT purities highet than 20%, and even more preferably HT purities higher than 25% are expected after elution. R/W values are in the range of 5-150 g/L, and more preferably in the range of 10-100 g/L.

Surprisingly, it is observed that the use of the resin with its neutral functional groups (uncharged) has advantages over the use of protonated groups (charged). Particularly, the use of neutral functional groups leads to higher HT adsorption yields in the resin, reducing the resin utilization for the same amount of processed extract compared to protonated resins at the same adsorption conditions. Additionally, it is observed that a resin used with neutral functional groups leads to higher purities than the same resin with its protonated functional groups. Another benefit of using neutral functional groups is that the resin does not require an acid activation stage, which reduces process times, process costs and chemical usage.

Batch or a continuous systems can be used to carry out the process. Continuous systems are preferred, as they reduce process times for the same amount of resin used. Additionally, part of the volume collected at the system outlet, which has a low HT purity, can be easily discarded in continuous systems to further increase the purity of the extract. On the other hand, batch systems would require multiple desorption steps to obtain a satisfactory similar result. For continuous systems, the operating flows for steps (b) and (c) should be in the range of 0,5 to 10 bed volumes per hour, and more preferably in the range of 1 to 8 bed volumes per hour.

Surprisingly, hot water initially elutes mainly compounds other than HT when continuous systems are used, meaning that the eluant has a low HT purity at the beginning of the process. Therefore, higher purity can be achieved if the low HT purity eluant that initially leaves the continuous system is discarded. Preferably, fractions with purity lower than the purity of the initial extract are discarded. More preferably, fractions with purity less than 5% are discarded.

Optionally, the resin obtained after elution of HT in step (c) can be regenerated to be reused. Preferably, the resin can be regenerated using an alkaline aqueous solution at a temperature less than 60°C. More preferably, the resin can be regenerated with an aqueous solution containing 1-5% NaOH, KOH, Ca(OH)₂, Na₂CO₃, NH₄OH or mixtures thereof at a temperature below 60°C.

Optionally, the eluted extract obtained from step (c) which is purified in HT can be concentrated for the removal of water. For the concentration, any method known from the state of the art can be used, such as falling film evaporation, rising film evaporation, scraped film evaporation, nanofiltration, reverse osmosis, evaporation, among others.

Optionally, the eluted extract obtained from step (c) or its concentrated product can be dried to obtain a powder. The drying method can be any known in the state of the art such as spray drying and lyophilization.

### APLICATION EXAMPLES

### Example 1

An extract with initial HT concentration of 0,9 g/L, 8,29% solids, HT purity of 1,09% (dry basis, d.b) and HPLC purity of 35,02% was treated using a weak base anion exchange resin. The resin was neutral or activated using acetic acid depending on the experiment. The tests were performed using a Dowex 66 resin (styrene-DVB matrix). The tests were carried out by adjusting the pH of the extract at 4,5 using NaOH. In each test, during the adsorption cycle, 3,73 g of resin were contacted with 30 mL of extract in an 80 mL flask for 2 hours at 25°C and constant agitation using an incubator (150 RPM). These conditions mean a resin load of 7,2 mg of HT/g of the resin. Then, the resin with the extract was filtered using grade 1 filter paper to recover the resin, then the resin was desorbed. Desorptions were carried out with distilled water at 25°C and 80°C using resin/water ratios (R/W) of 34 and 12 (weight/weight). The results of the tests are shown below.

| | **Mode** | **T (°C)** | **R/W** | **HT adsorbed (%)** | **Total HT (%)¹** | **Purity (%)²** | **dP³** | **Purity C (%)⁴** |
|---|---|---|---|---|---|---|---|---|
| **1** | Neutral | 25 | 34 | 78,4 | 22,9 | 10,2 | 9,3 | 62,6 |
| **2** | Neutral | 80 | 34 | 82,5 | 55,8 | 13,7 | 12,5 | 67,5 |
| **3** | Neutral | 25 | 12 | 80,4 | 28,5 | 10,4 | 9,5 | 45,6 |
| **4** | Neutral | 80 | 12 | 82,0 | 65,6 | 13,3 | 12,2 | 65,4 |
| **5** | Acid | 25 | 34 | 66,9 | 30,2 | 10,0 | 9,2 | 70,6 |
| **6** | Acid | 80 | 34 | 66,8 | 52,5 | 11,7 | 10,7 | 69,6 |
| **7** | Acid | 25 | 12 | 69,2 | 38,5 | 10,8 | 9,8 | 44,5 |
| **8** | Acid | 80 | 12 | 71,2 | 61,2 | 10,9 | 10,0 | 61,0 |

| | | | | | | | | |
|---|---|---|---|---|---|---|---|---|
| 1 Total recovery of HT during the process. 2 Purity measured as HT content relative to the total solids or dry basis purity (d.b). 3 Increase in purity relative to the extract. 4 Chromatographic purity expressed as the HT area relative to the total area. | | | | | | | | |

Comparing the results obtained with the resin in a neutral state (tests 1 to 4) with those obtained with the resin activated with acetic acid (tests 5-8), it can be observed that higher amount of HT is adsorbed in the neutral state. Values between 78,4% and 82,5% were observed for neutral resin, while valued ranged from 66,8% to 71,2% for the activated resin. On the other hand, for both resin modes, it is observed that increasing the water temperature from 25°C to 80°C improves the HT recovery with values between 1,6 and 2,4 times higher. Unexpectedly, using the resin in neutral mode led to higher purity than using the resin in acid mode at the same R/W and at 80°C. Consequently, the maximum purity value was 13,7% in test 2.

In order to compare previous results with using a strong anion exchange resin, tests were carried out under the same conditions using a strong anion exchange resin (IRA900Cl) treated with HCl as it is done in the state of the art. The following advantages are observed when using a weak base anion resin over a strong anion resin:
a) The weak base anion exchange resin has a HT adsorption capacity up to 28% higher than the strong anion exchange resin.
b) Higher purities are obtained by using a weak base anion resin compared to a strong anion resin.
c) Using hot water as eluent reduces the purity of the purified extracts when a strong anion resin is used, while using hot water improves the purity when a weak base anion resin was used.

### Example 3: Comparison of resins with different matrixes

To evaluate the effect of the resin matrix on the process, a process evaluation was carried out using Dowex 66 (polystyrene-DVB matrix) and IRA 67 (acrylic matrix) resins. An extract with initial HT concentration of 0,82 g/L, 4,27% solids, HT purity of 1,92% (d.b) and HPLC purity of 46,31% was used. The tests were performed by adjusting the pH of the extract at 4,5 using NaOH. In each test, during the adsorption cycle, 3,53 g of resin were contacted with 30 mL of extract in an 80 mL flask for 2 hours at 25°C and constant agitation using an incubator (150 RPM). The desorptions were carried out with water at 80°C using resin/water ratios of 13 (w/w). Additionally, IRA 67 resin previously activated with acetic acid was also evaluated as suggested in the state of the art. The results of the tests are shown below.

| | **Resin** | **Mode** | **HT adsorbed (%)** | **Total HT (%)** | **Purity (%)** | **dP** | **Purity C (%)** |
|---|---|---|---|---|---|---|---|
| **13** | Dowex 66 | Neutral | 80,2 | 68,8 | 20,8 | 11,5 | 61,09 |
| **14** | IRA 67 | Neutral | 81,4 | 7,9 | 3,5 | 1,9 | 4,11 |
| **15** | IRA 67 | Acid | 22,1 | 13,9 | 5,4 | 3 | 22,95 |

The results show that a lower amount of HT is desorbed from an acrylic-based resin, such as IRA 67, by using hot water, with a maximum value of 13,9%. Additionally, a maximum purity of 5,4% is obtained by using IRA 67 resin, which is 3,8 times lower than the purity obtained by using Dowex 66 resin. On the other hand, the conditioning of the IRA 67 resin with acetic acid significantly impairs the HT adsorption capacity in the resin, leading to an adsorption value (HT adsorbed) approximately 3,6 times lower than those obtained using the IRA 67 and Dowex 66 resins in a neutral mode.

Additionally, the effect of the pH of the extract in the purification process was evaluated because olive waste extracts destined to HT recovery are generally acidic to avoid the decomposition of HT; and also because previous process stages in which it has been shown in the literature that the use of acids allows the HT concentration to be increased. To evaluate the effect of the pH of the extract on the purification process, the previously described extract was adjusted to pH 2,2 using HCl. The results at pH 2,2 are presented below.

| | **Resin** | **Mode** | **HT adsorbed (%)** | **Total HT (%)** | **Purity (%)** | **dP** | **Purity C (%)** |
|---|---|---|---|---|---|---|---|
| **16** | Dowex 66 | Neutral | 75,4 | 62,9 | 21,3 | 11,1 | 80,06 |
| **17** | IRA 67 | Neutral | 40,0 | 15,5 | 13,7 | 7,1 | 75,35 |
| **18** | IRA 67 | Acid | 18,6 | 10,6 | 26,2 | 13,6 | 68,88 |

It can be observed that a reduction in pH has a significant effect on the behavior of IRA 67 resin, while the behavior of Dowex 66 resin slightly changes. Particularly for IRA 67, it is observed that when the pH of the extract is reduced, the HT adsorbed in a neutral mode is reduced from 81,4% to 40,0%, while for Dowex 66 the change is much smaller, shifting from 80,2% to 75,4%. Therefore, Dowex 66 allows a much more robust process related to the pH conditions of the extract to be treated.

On the other hand, a significant change is observed in the purity of the extracts obtained using IRA 67, where a maximum purity of 26,2% is reached with the acid mode resin. In this line, although the extract purities are higher in test 18 regarding all the previous tests (1 to 17), the HT recovery is considerably lower than other tests, reaching only 10,6%, which negatively affects the capital and operating costs of the process.

Finally, the effect of using acidified water with 0,05 M acetic acid on the desorption process was evaluated, obtaining the results below:

| | **Resin** | **Mode** | **HT adsorbed (%)** | **Total HT (%)** | **Purity (%)** | **dP** | **Purity C (%)** |
|---|---|---|---|---|---|---|---|
| **19** | Dowex 66 | Neutral | 79,5 | 59,4 | 18,9 | 10,4 | 71,5 |

The results show that the use of acidified water still allows to obtain extracts with a HT purity >10% and recovery >50%.

### Example 4: Comparison of nonionic adsorption resins and effect of temperature

An extract with initial HT concentration of 1,06 g/L, 7,64% solids, HT purity of 1,38% (d.b) and HPLC purity of 43,8%. The tests were carried out by adjusting the pH of the extract at 4,5 using HCl. In each test, during the adsorption cycle, 4,5 g of resin were contacted with 30 mL of the extract in an 80 mL flask for 2 hours at 25°C and constant agitation using an incubator. The desorptions were carried out with water using ratios resin/water of 13 (weight/weight). Dowex 66 resin was used in the neutral state.

| | **Resin** | **Mode** | **HT adsorbed (%)** | **Total HT (%)** | **Purity (%)** | **dP** | **Purity C (%)** |
|---|---|---|---|---|---|---|---|
| **20** | Dowex 66 | 60 | 85,9 | 60,3 | 18,4 | 13,3 | 72,7 |
| **21** | Dowex 66 | 70 | 85,9 | 67,6 | 16,6 | 12,0 | 72,1 |
| **22** | Dowex 66 | 80 | 85,7 | 69,0 | 17,3 | 12,5 | 72,5 |
| **23** | Dowex 66 | 90 | 85,9 | 72,3 | 12,9 | 9,3 | 71,3 |
| **24** | XAD4 | 80 | 76,6 | 68,3 | 8,2 | 5,9 | 60,4 |

In the case of Dowex 66 resin, the results show that purity values higher than 10% with HT recoveries higher than 50% are obtained in the temperature range between 60-90°C. Additionally, it is observed that an increase in temperature leads to an increase in recovery and a decrease in purity.

On the other hand, comparing the results using XAD4 with those obtained with Dowex 66 at 80°C, it can be observed that even when the recovery using both resins is similar (68,3% vs 69,0%), the purity obtained using Dowex 66 is 2,1 times higher, and the XAD4 resin does not reach a 10% purity.

### Example 5: Purification of a commercial extract

A commercial powder extract with HT purity of 4,07% was dispersed in water, obtaining a solution with 0,76 g/L of HT. The aqueous solution was purified using neutral Dowex 66 resin by contacting 3,5 g of resin with 30 mL of aqueous solution for 2 h at 25°C and constant stirring in an incubator (150 RPM). Then, the resin was desorbed using water at 80°C and a resin/water ratio of 13 for 2 hours. A final HT extract containing 26,3% with a total HT recovery of 58% was obtained from the process.

### Example 6: Continuous process using weak base ionic resins

An extract with initial HT concentration of 0,98 g/L, 8,95% solids and HT purity of 1,09% (d.b) was processed in a continuous bed loaded with 46,4 g of Dowex 66 resin in a neutral mode. To load the resin, 460 mL of extract at pH 4,5 were passed through the resin at a flow rate of 4 VL/h adsorbing up to 76,15% HT (7,4 mg HT/g of the resin) with a purity in the adsorbed 14,15%.

Once the adsorption process was finished, the resin was desorbed using 1,03 L of water at 80°C and a flow rate of 4 VL/h. The following table shows the amount of HT recovered and the purity of the accumulated extract at different times during the desorption process.

| **Fractions** | **Time (min)** | **HT Recovery (%)** | **HT Purity (%)** |
|---|---|---|---|
| | 0 | 0 | - |
| 1 | 7 | 8,3 | 1,6 |
| 2 | 18 | 23,6 | 16,9 |
| 3 | 28 | 37,8 | 36,5 |
| 4 | 40 | 48,8 | 39,5 |
| 5 | 53 | 57,5 | 45,9 |
| 6 | 78 | 70,2 | 41,2 |
| 7 | 130 | 85,7 | 33,8 |
| 8 | 160 | 88,8 | 20,1 |
| 9 | 200 | 90,5 | 19,2 |
| 10 | 220 | 91,1 | 19,2 |

The cumulated fractions have a total HT purity of 7,7% with a 91,1% of HT recovery from the adsorbed (69,2% total recovery), which shows an improvement of approximately 7 times of the HT purity of the extract. On the other hand, if the first two fractions are discarded due to their low HT concentration (particularly the first fraction) and only the remaining fractions are collected as product, an extract with 32,5% HT is obtained, and a recovery of 67,5% of the HT adsorbed (51,4% total recovery) is reached, which shows an improvement in approximately 29,8 times the HT purity of the extract.

As comparison, the test was carried out under the same conditions described above, but using a strong anion exchange resin (IRA 900CI) treated with HCl as is performed in the state of the art. It was observed that the weak base anion exchange resin has a higher HT adsorption capacity than the strong anion exchange resin, and a higher purity is obtained by using a weak base anion exchange resin as described in this invention.

## Claims

1. A process for obtaining concentrated hydroxytyrosol (HT) extracts using as raw material extracts with low concentration (less than 5% HT d.b) derived from the olive industry, without the use of organic solvents, that comprises the following steps:
a) adjusting the pH of an extract with low HT concentration to a pH value less than 7 using a pH regulator;
b) contacting the extract with low HT concentration with a weak base anion exchange resin of a polystyrene, styrene, divinylbenzene, polydivinylbenzene, polystyrene-DVB, or styrene-DVB matrix;
c) eluting the resin using hot water at a temperature between 50-100°C to obtain a purified HT extract.

2. The process according to claim 1, wherein the step (a) the pH regulator is selected from NaOH, KOH, Ca(OH)₂, Mg(OH)₂, Na₂CO₃, K₂CO₃, NaHCO₃, KHCO₃, NH₄OH, CaOH, MgO, HCl, acetic acid, citric acid, ascorbic acid, butyric acid, lactic acid, propionic acid, tartaric acid, oxalic acid, phosphoric acid, sulfuric acid, malic acid, succinic acid, sodium lactate, potassium lactate, sodium acetate, potassium acetate, sodium citrate, potassium citrate, sodium tartrate, potassium tartrate, sodium phosphate, potassium phosphate, sodium sulfate, potassium sulfate, malate sodium, potassium malate, or a mixture of them.

3. The process according to claim 1, wherein the resin used in step (b) has its functional groups either neutral or protonated.

4. The process according to claim 3, wherein the resin used in step (b) has its functional groups protonated.

5. The process according to claim 4, wherein any acid or mixture of acids selected from hydrochloric acid, acetic acid, citric acid, ascorbic acid, butyric acid, lactic acid, propionic acid, tartaric acid, oxalic acid, phosphoric acid, sulfuric acid, malic acid, succinic acid can be used to protonate the functional groups of the resin.

6. The process according to claim 3, wherein the resin used in step (b) has neutral functional groups.

7. The process according to claim 6, wherein any base or mixture of bases selected from NaOH, KOH, Ca(OH)₂, Na₂CO₃, NH₄OH can be used to obtain neutral functional groups.

8. The process according to claim 1, wherein in the step (c), an acid or mixture of acids can optionally be added to prevent the degradation of the hydroxytyrosol.

9. The process according to claim 8, wherein the acid or mixture of acids are selected from hydrochloric acid, acetic acid, citric acid, ascorbic acid, butyric acid, lactic acid, propionic acid, tartaric acid, oxalic acid, phosphoric acid, acid sulfuric, malic acid, succinic acid.

10. The process according to claims 1 to 9, wherein a batch or continuous mode system can be used.

11. The process according to claim 10, wherein a continuous system is preferably used.

12. The process according to claims 1 to 11, wherein the purified extract rich in hydroxytyrosol is concentrated to remove water.

13. The process according to claims 1 to 12, wherein the purified extract rich in hydroxytyrosol is dried to obtain a powder product.

14. A product obtained based on the process according to claim 12.

15. A product obtained based on the process according to claim 13.

## Patentansprüche

1. Verfahren zur Gewinnung von konzentrierten Hydroxytyrosol (HT)-extrakten unter Verwendung, als Rohstoff, von Extrakten mit niedriger Konzentration (weniger als 5% HT auf trockener Basis) abgeleitet von der Olivenindustrie, ohne die Verwendung von organischen Lösungsmitteln, welches die folgenden Schritte umfasst:
a) das Einstellen des pH-Wertes eines Extrakts mit niedriger HT-Konzentration auf einen pH-Wert von weniger als 7 unter Verwendung eines pH-Regulators;
b) das Inkontaktbringen des Extrakts mit niedriger HT-Konzentration mit einem schwach basischen Anionenaustauscherharz einer Polystyrol-, Styrol-, Divinylbenzol-, Polydivinylbenzol-, Polystyrol-DVB- oder Styrol-DVB-Matrix;
c) das Eluieren des Harzes unter Verwendung von heißem Wasser bei einer Temperatur zwischen 50-100°C, um einen gereinigten HT-Extrakt zu gewinnen.

2. Verfahren nach Anspruch 1, wobei in Schritt (a) der pH-Regulator aus NaOH, KOH, Ca(OH)₂, Mg(OH)₂, Na₂CO₃, K₂CO₃, NaHCO₃, KHCO₃, NH₄OH, CaOH, MgO, HCl, Essigsäure, Zitronensäure, Ascorbinsäure, Buttersäure, Milchsäure, Propionsäure, Weinsäure, Oxalsäure, Phosphorsäure, Schwefelsäure, Apfelsäure, Bernsteinsäure, Natriumlactat, Kaliumlactat, Natriumacetat, Kaliumacetat, Natriumcitrat, Kaliumcitrat, Natriumtartrat, Kaliumtartrat, Natriumphosphat, Kaliumphosphat, Natriumsulfat, Kaliumsulfat, Natriummalat, Kaliummalat oder einer Mischung derselben ausgewählt wird.

3. Verfahren nach Anspruch 1, wobei das in Schritt (b) verwendete Harz dessen funktionellen Gruppen entweder neutral oder protoniert aufweist.

4. Verfahren nach Anspruch 3, wobei das in Schritt (b) verwendete Harz dessen funktionellen Gruppen protoniert aufweist.

5. Verfahren nach Anspruch 4, wobei jede Säure oder Mischung von Säuren ausgewählt aus Salzsäure, Essigsäure, Zitronensäure, Ascorbinsäure, Buttersäure, Milchsäure, Propionsäure, Weinsäure, Oxalsäure, Phosphorsäure, Schwefelsäure, Apfelsäure, Bernsteinsäure dazu verwendet werden kann, die funktionellen Gruppen des Harzes zu protonieren.

6. Verfahren nach Anspruch 3, wobei das in Schritt (b) verwendete Harz neutrale funktionelle Gruppen aufweist.

7. Verfahren nach Anspruch 6, wobei jede Basis oder Mischung von Basen ausgewählt aus NaOH, KOH, Ca(OH)₂, Na₂CO₃, NH₄OH dazu verwendet werden kann, neutrale funktionelle Gruppen zu gewinnen.

8. Verfahren nach Anspruch 1, wobei in Schritt (c) eine Säure oder Mischung von Säuren wahlweise hinzugefügt werden kann, um die Zersetzung des Hydroxytyrosols zu verhindern.

9. Verfahren nach Anspruch 8, wobei die Säure oder die Mischung von Säuren aus Salzsäure, Essigsäure, Zitronensäure, Ascorbinsäure, Buttersäure, Milchsäure, Propionsäure, Weinsäure, Oxalsäure, Phosphorsäure, Schwefelsäure, Apfelsäure, Bernsteinsäure ausgewählt werden.

10. Verfahren nach den Ansprüchen 1 bis 9, wobei ein System im diskontinuierlichen oder kontinuierlichen Modus verwendet werden kann.

11. Verfahren nach Anspruch 10, wobei vorzugsweise ein kontinuierliches System verwendet wird.

12. Verfahren nach den Ansprüchen 1 bis 11, wobei der gereinigte, reich an Hydroxytyrosol, Extrakt konzentriert wird, um Wasser zu entfernen.

13. Verfahren nach den Ansprüchen 1 bis 12, wobei der gereinigte, reich an Hydroxytyrosol, Extrakt getrocknet wird, um ein Pulverprodukt zu gewinnen.

14. Produkt gewonnen basierend auf dem Verfahren nach Anspruch 12.

15. Produkt gewonnen basierend auf dem Verfahren nach Anspruch 13.

## Revendications

1. Procédé pour obtenir des extraits concentrés d'hydroxytyrosol (HT) en utilisant comme matière première des extraits à faible concentration (moins de 5% de HT d.b) dérivés de l'industrie oléicole, sans utiliser des solvants organiques, comprenant les étapes suivantes :
a) ajuster le pH d'un extrait à faible concentration en HT à une valeur de pH inférieure à 7 en utilisant un régulateur de pH ;
b) mettre l'extrait à faible concentration en HT en contact avec une résine échangeuse d'anions à base faible d'une matrice de polystyrène, styrène, divinylbenzène, polydivinylbenzène, polystyrène-DVB ou styrène-DVB ;
c) éluer la résine en utilisant de l'eau chaude à une température comprise entre 50-100°C pour obtenir un extrait de HT purifié.

2. Procédé selon la revendication 1, dans lequel, dans l'étape (a), le régulateur de pH est choisi parmi NaOH, KOH, Ca(OH)₂, Mg(OH)₂, Na₂CO₃, K₂CO₃, NaHCO₃, KHCO₃, NH₄OH, CaOH, MgO, HCl, l'acide acétique, l'acide citrique, l'acide ascorbique, l'acide butyrique, l'acide lactique, l'acide propionique, l'acide tartrique, l'acide oxalique, l'acide phosphorique, l'acide sulfurique, l'acide malique, l'acide succinique, le lactate de sodium, le lactate de potassium, l'acétate de sodium, l'acétate de potassium, le citrate de sodium, le citrate de potassium, le tartrate de sodium, le tartrate de potassium, le phosphate de sodium, le phosphate de potassium, le sulfate de sodium, le sulfate de potassium, le malate de sodium, le malate de potassium ou un mélange de ceux-ci.

3. Procédé selon la revendication 1, dans lequel la résine utilisée dans l'étape (b) présente ses groupes fonctionnels soit neutres soit protonés.

4. Procédé selon la revendication 3, dans lequel la résine utilisée dans l'étape (b) présente ses groupes fonctionnels protonés.

5. Procédé selon la revendication 4, dans lequel tout acide ou mélange d'acides choisis parmi l'acide chlorhydrique, l'acide acétique, l'acide citrique, l'acide ascorbique, l'acide butyrique, l'acide lactique, l'acide propionique, l'acide tartrique, l'acide oxalique, l'acide phosphorique, l'acide sulfurique, l'acide malique, l'acide succinique, peut être utilisé pour protoner les groupes fonctionnels de la résine.

6. Procédé selon la revendication 3, dans lequel la résine utilisée dans l'étape (b) présente des groupes fonctionnels neutres.

7. Procédé selon la revendication 6, dans lequel toute base ou mélange de bases choisies parmi NaOH, KOH, Ca(OH)₂, Na₂CO₃, NH₄OH, peut être utilisé pour obtenir des groupes fonctionnels neutres.

8. Procédé selon la revendication 1, dans lequel, dans l'étape (c), un acide ou mélange d'acides peut optionnellement être ajouté pour empêcher la dégradation de l'hydroxytyrosol.

9. Procédé selon la revendication 8, dans lequel l'acide ou mélange d'acides sont choisis parmi l'acide chlorhydrique, l'acide acétique, l'acide citrique, l'acide ascorbique, l'acide butyrique, l'acide lactique, l'acide propionique, l'acide tartrique, l'acide oxalique, l'acide phosphorique, l'acide sulfurique, l'acide malique, l'acide succinique.

10. Procédé selon les revendications 1 à 9, dans lequel un système en mode discontinu ou continu peut être utilisé.

11. Procédé selon la revendication 10, dans lequel un système continu est préférablement utilisé.

12. Procédé selon les revendications 1 à 11, dans lequel l'extrait purifié riche en hydroxytyrosol est concentré pour éliminer l'eau.

13. Procédé selon les revendications 1 à 12, dans lequel l'extrait purifié riche en hydroxytyrosol est séché pour obtenir un produit en poudre.

14. Produit obtenu sur la base du procédé selon la revendication 12.

15. Produit obtenu sur la base du procédé selon la revendication 13.
